# EUROPÄISCHE PATENTSCHRIFT

(11) **EP 3 395 380 B1**
(45) Veröffentlichungstag und Bekanntmachung des Hinweises auf die Patenterteilung: **22.07.2020**
(21) Anmeldenummer: 18167498.7
(22) Anmeldetag: 16.04.2018
(51) Int. Cl.: A61L 29/06

(54) **BALLOON FÜR EINEN BALLONKATHETER SOWIE VERFAHREN ZUR HERSTELLUNG DES BALLONS**
BALLOON FOR A BALLOON CATHETER AND METHOD FOR PRODUCTION OF SUCH A BALLOON
BALLONNET POUR UN CATHÉTER À BALLONNET AINSI QUE SON PROCÉDÉ DE FABRICATION

(30) Priorität: 24.04.2017 EP 17167689
(43) Veröffentlichungstag der Anmeldung: 31.10.2018
(73) Patentinhaber: BIOTRONIK AG, 8180 Bülach (CH)
(72) Erfinder: Schwitzer, Alwin, 8180 Bülach (CH)
(74) Vertreter: Randoll, Sören

(56) Entgegenhaltungen:
- EP-A1- 1 598 090
- EP-A1- 2 149 387
- EP-A1- 2 446 917
- EP-A1- 2 628 438
- WO-A1-02/058913
- DE-A1- 10 215 462
- DE-T2- 60 221 388
- DE-U1-202012 102 061

## Beschreibung

Die vorliegende Erfindung betrifft einen Ballon, insbesondere ein Polymer für einen Ballon, einen Ballonkatheter sowie ein Verfahren zur Herstellung des Ballons.

Solche Ballone bzw. Ballonkatheter werden z. B. für das Aufweiten von krankhaft verengten Gefäßen im Körper eines Patienten (Ballondilatation) oder für das Setzen von Gefäßwandstützen (sogenannte Stents) verwendet.

Derartige Ballone sind z. B. aus der US 2014/0116606 A1 bekannt. Darin wird z. B. ein Hochdruckballon beschrieben, der aus mindestens zwei Schichten (Doppelmembran) besteht.

Ein solcher Ballon kann grundsätzlich z. B. aus einem coextrudierten Schlauch mit zwei oder mehr Schichten gefertigt sein. Es besteht auch die Möglichkeit, dass zur Herstellung eines solchen mehrlagigen Ballons eine entsprechende Anzahl an Schläuchen vor dem Ballonformen ineinander geschoben wird. Weiterhin kann auch in einer sonstigen Weise ein äußerer Ballon um einen inneren Ballon herum erzeugt werden.

Allerdings sind die vorgenannten Prozesse regelmäßig vergleichsweise aufwändig. Weiterhin sind mehrlagige Ballonhüllen aufgrund der größeren Gesamtwandstärke nachteilig im Hinblick auf die Platzierung des Katheters (so genannte Deliverability) am Zielort.

Die WO02058913 beschreibt Katheter-Ballons für u.a. Gefäßaufweitung und Stent-Setzen, hergestellt aus schlauchförmigen Rohlingen durch radiale Expansion. Die Ballonmaterialien umfassen bevorzugt Polyamide, Polyurethane oder deren Blockcopolymere. Als Polyamid sind unter anderem Nylon 6, Nylon 6/6, Nylon 6/10, Nylon 6/12, Nylon 9, Nylon 11 und Nylon 12 genannt.

Hiervon ausgehend liegt der vorliegenden Erfindung daher die Aufgabe zugrunde, einen Ballon bzw. einen Ballonkatheter bzw. ein Verfahren zur Herstellung eines solchen Ballons zu schaffen, der bzw. das im Hinblick auf die vorgenannte Problematik verbessert ist.

Diese Aufgabe wird durch einen Ballon, insbesondere durch einen Hochdruckballon, mit den Merkmalen des Anspruchs 1, einen Ballonkatheter mit den Merkmalen des Anspruchs 2 sowie durch ein Verfahren mit den Merkmalen des Anspruchs 4 gelöst. Vorteilhafte Ausgestaltungen dieser Aspekte der vorliegenden Erfindung sind in den entsprechenden Unteransprüchen angegeben und werden nachfolgend beschrieben.

Gemäß Anspruch 1 wird ein Ballon, insbesondere ein Hochdruckballon für einen Ballonkatheter offenbart, wobei der Ballon eine aufblasbare sowie einlagige Ballonhülle aufweist, die einen Innenraum umgibt und durch Befüllen des Innenraumes mit einem Fluid dilatierbar ist, wobei erfindungsgemäße die Ballonhülle aus Polyamid 6.12 gebildet ist oder Polyamid 6.12 aufweist. Die Ballonhülle kann weitere Stoffe aufweisen, z. B. Verstärkungsstoffe, z. B. Nanocomposites zur weiteren Erhöhung des Platzdrucks des Ballons. Das Ballonmaterial ist allerdings vorzugsweise frei von Farbstoffen oder anderen Zusätzen, die eine sondierte Bildgebung unterstützen.

Bei dem Stoff Polyamid 6.12 handelt es sich um Poly-(hexamethylendodecandiamid) mit der CAS-Nummer 26098-55-5. Polyamid 6.12 ist also ein Polyamid mit den Monomerbausteinen Hexamethylendiamin und Dodecandisäure, im Gegensatz zu dem "state of the art" Ballonmaterial Polyamid 12 mit dem Monomerbaustein Laurinlactam. Das erfindungsgemäße Ballonmaterial Polyamid 6.12 ermöglicht mit Vorteil die Herstellung eines Hochdruckballons mit einem RBP (Rated Burst Pressure, zu Deutsch: Nennberstdruck), der oberhalb von 20 bar liegt sowie eine Fatigue-Beständigkeit (20 Zyklen) bei RBP aufweist. Das besagte Ballonmaterial ermöglicht weiterhin mit Vorteil vergleichsweise dünne Ballonwandstärke, so dass der gefaltete Ballon einen geringen Durchmesser aufweist und eine Eignung für ein Introducersystems mit entsprechend kleinen Frenchgrößen vorliegt. Weiterhin wird hierdurch eine bessere Trackability ermöglicht. Zudem erlaubt das erfindungsgemäße Ballonmaterial eine einfache Herstellung des Ausgangsschlauches (Ballonschlauches) für die Formung des Ballons. Insbesondere wird keine Coextrusion sowie kein Ineinanderschieben von unterschiedlichen Schläuchen zur Herstellung benötigt. Die Ballonformung kann weiterhin mit Vorteil auf herkömmlichen Ballonformanalgen in einem Schritt stattfinden; eine zweifache Ballonformung ist nicht notwendig. Schließlich erlaubt das erfindungsgemäße Ballonmaterial mit Vorteil ein Verschweißen mit den restlichen Katheter-Komponenten.

Der Umstand, dass die erfindungsgemäße Ballonhülle einlagig ausgebildet ist, bedeutet insbesondere, dass die Ballonhülle aus einer einzigen Materiallage gebildet ist, die eine äußerste, nach außen gewandte Außenseite sowie eine innerste, dem Innenraum zugewandte Innenseite der Ballonhülle bildet.

Das erfindungsgemäße Material erlaubt mit Vorteil ein Extrudieren des Materials, insbesondere zur Herstellung von schlauchförmigen Rohlingen sowie ein thermisches Umformen eines solchen Rohlings zur Ballonformung. Ein solchermaßen hergestellter Ballon ist weiterhin sterilisierbar sowie mit den restlichen Katheter-Komponenten verschweißbar, so dass die Hochdruckeigenschaften des Ballons auch auf den gesamten Katheter übertragbar sind.

Der Ballon, insbesondere der Hochdruckballon, gemäß Anspruch 1 ist dadurch gekennzeichnet, dass die Ballonhülle eine Dicke im Verhältnis zum nominalen Ballondurchmesser im Bereich von 5,0 µm/mm bis 7,5 µm/mm aufweist, bevorzugt ∼6,3 µm/mm.

Der erfindungsgemäße Ballon, insbesondere Hochdruckballon, der wie oben beschrieben eine aufblasbare und einlagige Ballonhülle aufweist, weist bei diesen Dicken gleichfalls vorteilhafter und überraschender Weise einen RBP oberhalb von 20 bar sowie eine Fatigue-Beständigkeit (20 Zyklen) bei RBP auf. Mit RBP wird auf den Druck verwiesen, der durch die Normen ISO 25539-1, ISO 25539-2 und ISO 10555-4 definiert ist. Im Sinne von FDA "Guidance for Industry and FDA Staff Class II Special Controls Guidance Document for Certain Percutaneous Transluminal Coronary Angioplasty (PTCA) Catheters; Guidance for Industry and FDA" ist der RBP (Rated Burst Pressure, zu Deutsch: Nennberstdruck) derjenige Druck den 99.9% der Ballone mit 95% Konfidenz ohne zu platzen überstehen.

Gemäß einem weiteren Aspekt der Erfindung wird ein Ballonkatheter offenbart, mit:
- einem Außenschaft mit einem distalen Endabschnitt,
- einen im Außenschaft geführten Innenschaft, der mit einem distalen Endabschnitt über den distalen Endabschnitt des Außenschaftes hinaussteht, sowie mit
- einem erfindungsgemäßen, hierin beschriebenen bzw. beanspruchten Ballons, wobei jenes Fluid zum Dilatieren des Ballons über den Außenschaft in den Innenraum der Ballonhülle einleitbar ist, und
- wobei die Ballonhülle mit einem proximalen Endabschnitt stoffschlüssig mit dem distalen Endabschnitt des Außenschaftes verbunden ist, und wobei die Ballonhülle mit einem distalen Endanschnitt mit dem distalen Endabschnitt des Innenschaftes stoffschlüssig verbunden ist.

Bevorzugt ist die Ballonhülle gemäß einer Ausführungsform im undilatierten Zustand in Längsfalten gelegt und schmiegt sich dabei insbesondere eng an den Innenkatheter an. Weiterhin ist gemäß einer Ausführungsform des erfindungsgemäßen Ballonkatheters vorgesehen, dass zur Herstellung der besagten stoffschlüssigen Verbindung bzw. Verbindungen der proximale Endabschnitt der Ballonhülle mit dem distalen Endabschnitt des Außenschaftes verschweißt ist, und/oder dass der distale Endabschnitt der Ballonhülle mit dem distalen Endabschnitt des Innenschaftes verschweißt ist.

Gemäß einem weiteren Aspekt der vorliegenden Erfindung wird schließlich ein Verfahren zur Herstellung eines Ballons für einen Ballonkatheter offenbart, wobei eine mittels Druckluft dilatierbare Ballonhülle des Ballons aus einem Material geformt wird, bei dem es sich um Polyamid 6.12 handelt oder das Polyamid 6.12 aufweist.

Gemäß des erfindungsgemäßen Verfahrens ist vorgesehen, dass zum Herstellen der Ballonhülle das Material zu einem einlagigen schlauchförmigen Rohling extrudiert wird, der einen Innenraum umschließt und sich entlang einer axialen Richtung erstreckt.

Schlauchförmig bedeutet hierbei insbesondere, dass der Rohling eine umlaufende, insbesondere zylindrische, Wandung aufweist, die den Innenraum des Rohlings umschließt, sowie zwei einander gegenüberliegenden Öffnungen, wobei ein umlaufender Randbereich der jeweiligen Öffnung den proximalen bzw. distalen Endabschnitt der herzustellenden Ballonhülle bildet.

Gemäß einer bevorzugten Ausführungsform des Verfahrens ist vorgesehen, dass der Rohling einer Streckung in der axialen Richtung unterzogen wird, z. B. durch ein Aufbringen einer axialen Streckkraft auf den extrudierten Rohling sowie durch ein Erhitzen des extrudierten Rohlings, so dass sich dieser in der axialen Richtung strecken kann, während die axiale Streckkraft aufrechterhalten wird. Hiernach wird der Rohling vorzugsweise abgekühlt.

Weiterhin ist gemäß einer Ausführungsform des Verfahrens vorgesehen, dass der extrudierte Rohling auf eine Temperatur im Bereich von 120°C bis 160°C, insbesondere 140°C, gebracht wird und radial zu der Form der Ballonhülle expandiert wird.

Weiterhin ist gemäß einer Ausführungsform des Verfahrens vorgesehen, dass das Expandieren ein Beaufschlagen des Innenraumes des Rohlings mit einem Druck im Bereich von 20 bar bis 50 bar, insbesondere 30 bar bis 35 bar, umfasst.

Gemäß einer Ausführungsform umfasst das Expandieren ein Einbringen des Rohlings in eine Form, ein Erhitzen der Form auf die besagte Temperatur und ein radiales Expandieren des gestreckten Schlauches unter Anwendung des besagten Drucks im Innenraum des Rohlings.

Nach erfolgter Formung der Ballonhülle wird die Form abgekühlt und der Ballon entnommen.

Weitere Merkmale und Vorteile der vorliegenden Erfindung sollen bei der nachfolgenden Figurenbeschreibung eines Ausführungsbeispiels der Erfindung anhand der Figuren erläutert werden. Es zeigt:
- Fig. 1: eine schematische Schnittdarstellung eines erfindungsgemäßen Ballons bzw. Ballonkatheters; und
- Fig. 2: eine schematische Darstellung der Herstellung eines erfindungsgemäßen Ballons.
- Fig. 3: die Feuchteaufnahme von Polyamiden (PA6, PA66, PA612, PA12),
- Fig. 4: den Zug-E-Modul von Polyamiden (PA66, PA6 und PA12),
- Fig. 5: zeigt die Ballon-Compliance eines Grilamid 2D20-Ballons im Vergleich zu einem konventionellen einschichtigen PA12-basierenden Hochdruck-Ballon,
- Fig. 6: Spannungs-Dehnungskurven (berechnet aus den Compliance-Kurven gemäß Figur 5 mit den Wandstärken der geformten Ballone),
- Fig. 7: den RBP der getesteten Ballone,
- Fig. 8: die Compliance getesteter Ballone,
- Fig. 9: die Ballon-Fatigue getesteter Ballone,
- Fig. 10: die für Polyamide charakteristische Amidgruppe (-HN-CO-), wie sie auch bei Peptiden vorhanden ist,
- Fig. 11: durch Wasserstoffbrückenbindung (siehe Pfeil) verbundene Amidgruppen gemäß Figur 10, die die thermischen und mechanischen Eigenschaften von Polyamiden begründen. Die Bindungsenergie der Wasserstoffbrückenbindung liegt bei etwa 20 kJ/mol. Sie wird erst bei hohen Belastungen gelöst und baut sich nach einer Verschiebung sofort wieder auf,
- Fig. 12: zeigt die Struktur von PA66,
- Fig. 13: zeigt die Struktur von PA6, und
- Fig. 14: zeigt die Struktur von PA12.

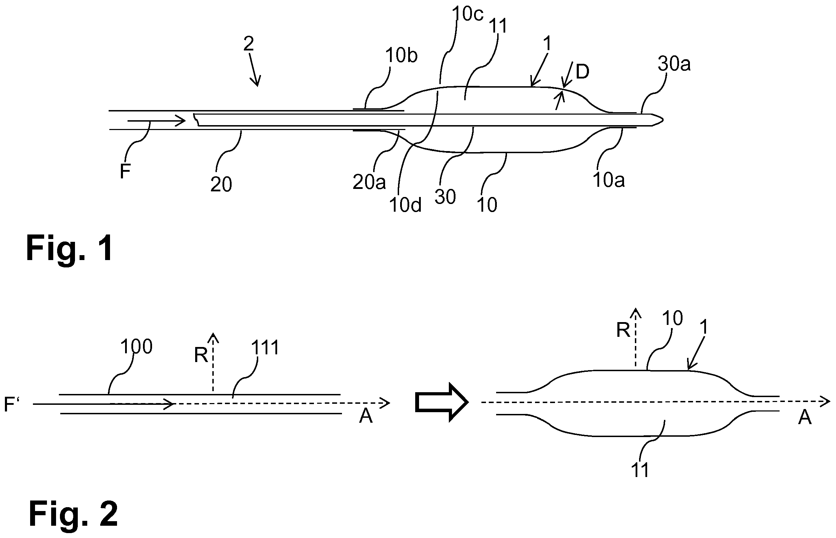

Figur 1 zeigt eine schematische Schnittansicht eines erfindungsgemäßen Ballons 1 bzw. Ballonkatheters 2. Der Ballon 1 weist eine aufblasbare sowie einlagige Ballonhülle 10 auf, die einen Innenraum 11 umgibt und durch Befüllen des Innenraumes 11 mit einem Fluid F dilatierbar bzw. aufblasbar ist. Erfindungsgemäß ist vorgesehen, dass die Ballonhülle 10 aus Polyamid 6.12 gebildet ist oder Polyamid 6.12 aufweist.

Erfindungsgemäß weist der Ballon 1 eine Dicke im Verhältnis zum nominalen Ballondurchmesser im Bereich von 5,0 µm/mm bis 7,5 µm/mm auf, bevorzugt 6,3 µm/mm.

Der Ballon 1 wird gemäß einem Aspekt der Erfindung als Ballon eines Ballonkatheters 2 eingesetzt. Dieser weist einen Außenschaft 20 mit einem distalen Endabschnitt 20a auf, sowie einen im Außenschaft 20 geführten Innenschaft 30, der mit einem distalen Endabschnitt 30a über den distalen Endabschnitt 20a des Außenschaftes 20 hinaussteht. Die Ballonhülle 10 ist nun mit einem proximalen Endabschnitt 10b stoffschlüssig mit dem distalen Endabschnitt 20a des Außenschaftes 20 sowie mit einem distalen Endabschnitt 10a mit dem distalen Endabschnitt 30a des Innenschaftes 30 stoffschlüssig sowie fluiddicht verbunden. Hierbei kann ein Fluid F zum Dilatieren des Ballons 1 über den Außenschaft (am Innenschaft vorbei) in den Innenraum 11 der Ballonhülle 10 eingeleitet werden. Die besagten stoffschlüssigen Verbindungen werden bevorzugt als Schweißverbindungen ausgeführt.

Figur 2 zeigt eine schemtische Darstellung einer Herstellung des Ballons 1. Hierbei wird zunächst zum Herstellen der Ballonhülle 10 das durch Polyamid 6.12 gebildete bzw. das Polyamid 6.12 aufweisende Material zu einem einlagigen schlauchförmigen (z. B. zylindrischen) Rohling 100 extrudiert, der einen Innenraum 111 umschließt und sich entlang einer axialen Richtung A erstreckt. Der Rohling 100 kann vor der Ballonformung einer Streckung in der axialen Richtung A unterzogen werden (z. B. durch Erhitzen und Aufbringen einer in der axialen Richtung A wirkenden Streckkraft).

Der ggf. gestreckte Rohling 100 wird anschließend auf eine Temperatur im Bereich von 120°C bis 160°C, insbesondere 140°C, gebracht und wird in der radialen Richtung R zu der Form der Ballonhülle 10 expandiert. Das Expandieren erfolgt vorzugsweise durch ein Beaufschlagen des Innenraumes 111 des Rohlings 100 mit einem Druck im Bereich von 20 bar bis 50 bar, insbesondere 30 bar bis 35 bar, z. B. indem ein Fluid F (z.B. Druckluft) mit einem entsprechenden Druck in den Innenraum 111 bzw. 11 eingeleitet wird (siehe auch oben).

Als Polyamid 6.12 kann z. B. Grilamid 2D20 des Herstellers EMS-CHEMIE AG oder Vestamid D16, Vestamid D18, Vestamid D22, oder Vestamid D26 des Herstellers EVONIK Industries oder weiteren Herstellern verwendet werden.

Gemäß einem Beispiel der Erfindung wurde ein einschichtiger Ballonschlauch aus Grilamid 2D20 extrudiert. Die Ballonformung erfolgte bei einer Temperatur von ∼140°C mit einem Blasdruck von ∼30 bar auf einer herkömmlichen Ballonformanlage, die z. B. eine Formung des Ballons in einer geschlossenen Form unter Druckbeaufschlagung von innen vorsieht.

Der solchermaßen hergestellte Ballon aus Grilamid 2D20 weist einen Durchmesser von 3.0 mm auf und erzielt einen RBP von 30bar mit einer Wanddicke D von 0,019 mm (6,3 µm/mm).

Ein Vergleichsballon aus Polyamid 12 (PA12) erzielte demgegenüber einen RBP von 24 bar mit doppelter Wandstärke von 0,040 mm. Das heißt, der erfindungsgemäß hergestellte Ballon hat bei 5% geringerer Ballonwandstärke D einen 6 bar höheren Platzdruck (RBP) auf Ballonstufe.

Weiterhin kann das erfindungsgemäß verwendete Polymer Polyamid 6.12 aufgrund der Herstellung aus Diamin und Dicarbonsäure im Vergleich zu PA12 oder daraus abgeleiteten Polymeren (z. B. PEBAX Typen) mit Vorteil keinen "Blooming"-Effekt zeigen. Bei Polyamiden, die aus Lactamen gebildet werden, z. B. Polyamid 12, kann nämlich nicht reagiertes Lactam im Polymer mit der Zeit aus dem geformten Teil an die Oberfläche migrieren. Bei Polyamiden, die aus Dicarbonsäuren und Diaminen gebildet werden, ist dies nicht möglich, da evtl. nicht reagierte Monomere als Salz in der Polymermatrix vorliegen.

Die erfindungsgemäßen Ballons können zudem leicht und kostengünstig aus einem einschichtig extrudierten Schlauch hergestellt werden.

Im Folgenden sollen weitere Beispiele der Erfindung sowie Vergleichsuntersuchungen detailliert vorgestellt werden, die die vorteilhafte Eignung von Polyamid 6.12 als Ballonmaterial bzw. Bestandteil eines Ballons belegen.

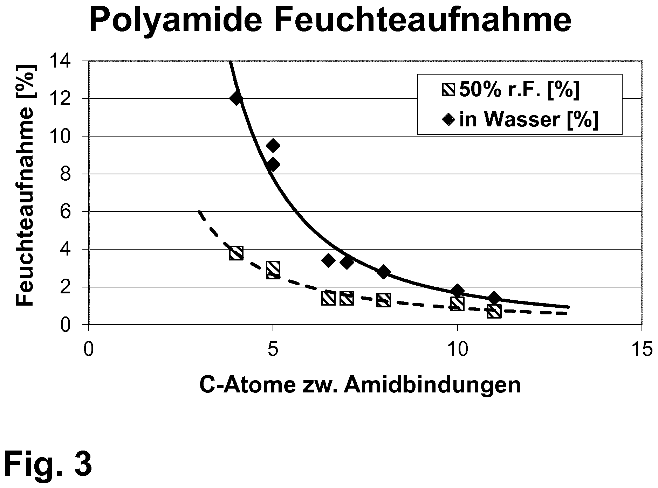

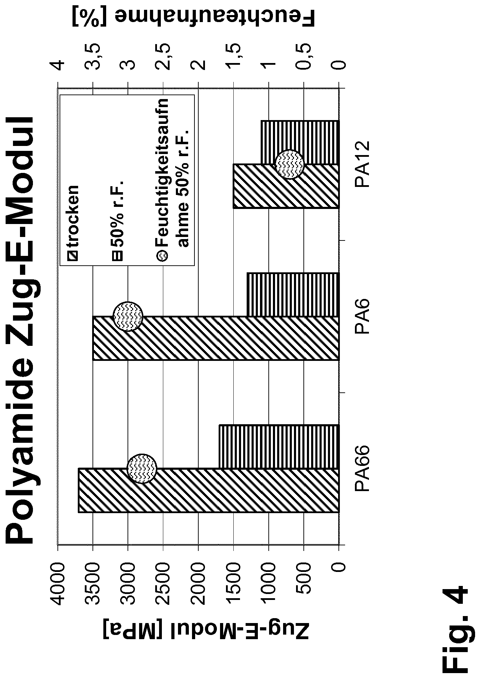

Die besondere Eignung von Polyamid 6.12 als Ballonmaterial liegt diesbezüglich u. a. auch in der vergleichsweise geringen Feuchtigkeitsaufnahme von Polyamid 6.12 (Fig. 3: PA6/12 8-C-Atome, PA6, PA66: 5 C-Atome). Dies ist bedeutsam, da Polyamide mit starker Feuchtigkeitsaufnahme einen starken Abfall der mechanischen Eigenschaften aufweisen. Diesbezüglich zeigt Fig. 4 das Zug-E-Modul weiterer Polyamide (PA66, PA6 und PA12).

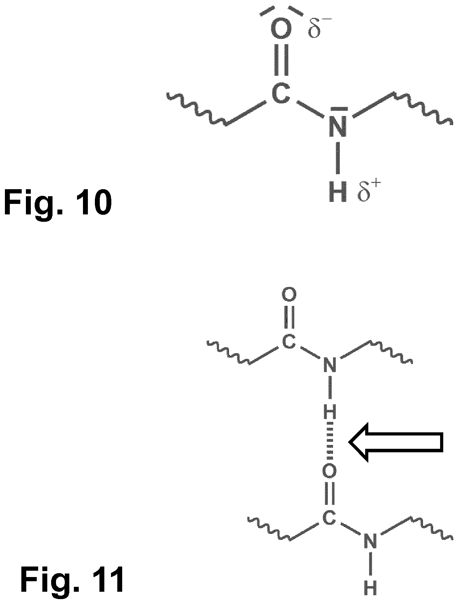

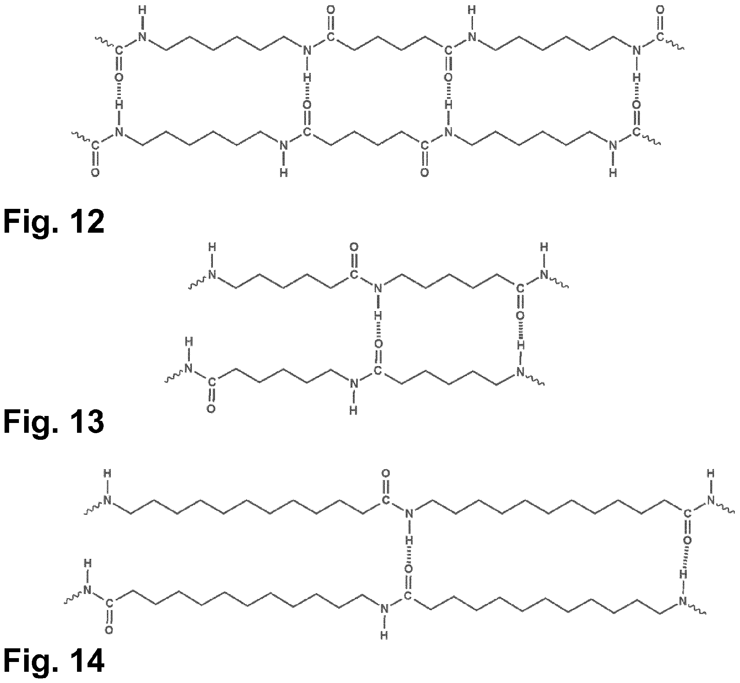

Je mehr Kohlenstoff-Atome zwischen den Amidbindungen vorhanden sind (siehe z. B. Figuren 10 bis 14), desto niedriger ist die Feuchtigkeitsaufnahme des betreffenden Polyamids. Weiterhin nimmt aber mit steigender Anzahl der Kohlenstoff-Atome zwischen den Amidbindungen der Zug-E-Modul und damit die mechanische Festigkeit/Steifigkeit des betreffenden Polyamids ab.

Polyamid 6.12 ist daher eine vorteilhafte Alternative zu den bestehenden Polyamiden (vgl. Fig. 3) für Ballone in insbesondere vaskulären Kathetern, da die vergleichsweise geringe Feuchtigkeitsaufnahme mit einer entsprechend geringen Reduktion der mechanischen Eigenschaften durch die Feuchtigkeitsaufnahme einhergeht und insbesondere einen höheren E-Modul als Polyamid 12 (PA12) aufweist.

Als Polyamid 6.12 wird im Folgenden insbesondere Grilamid 2D20® / EMS-Chemie oder Vestamid D® / Evonik betrachtet.

Wie oben bereits dargelegt, handelt es sich bei Polyamid 6.12 (PA612) um ein Polyamid, das aus 1,6-Hexandiamin und Dodecandisäure über Polykondensation herstellbar ist. Im Unterschied zu Polyamid 12, welches aus Laurinlactam hergestellt wird, Polyamid 6, das aus Caprolactam hergestellt wird oder Polyamid 66, welches aus 1,6-Hexandiamin und Adipinsäure polykondensiert wird.

Im Vergleich zu PA12 PEBAX 7033 und PA6/12 (Co-Polyamid aus Caprolactam und Laurinlactam, Eigenschaften sind abhängig vom Verhältnis der beiden Monomere) zeigt Polyamid 6.12 die folgenden Eigenschaften:

| | Grilamid 2D 20 | Grilamid L25 | PEBAX 7033 | PA6/12 Copolyamide |
|---|---|---|---|---|
| E-Modul konditioniert (50% r.F) / MPa | 1600 | 1100 | 384 | 400 - 550 |
| tensile strength @ break / MPa | 50 | 50 | 54 | |
| Elongation @ break / % | 16 | > 50 | > 350 | |
| shore D Härte | 81 | 70 | 61 | |
| | | | | |
| Polymer classification | PA612 | PA12 | PEBA | PA6/12 |

Insbesondere zeigt das Polyamid PA612 (Grilamid 2D20) nach einer Konditionierung (Wasseraufnahme durch Lagerung bei 50% relativer Feuchte) einen höheren E-Modul als PA12 oder PEBAX 7033, die im Stand der Technik als einschichtige Ballonmaterialien für Katheter eingesetzt werden.

Wie bereits dargelegt, kann aufgrund des Aufbaus des PA612 aus einem Diamin und einer Dicarbonsäure bei diesem Polyamid (im Unterschied zu PA12 oder den PA12 basierenden PEBAX Typen) kein freies Monomer vorhanden sein, welches durch Alterung aus dem Bauteil heraus migrieren kann (sogenanntes "Blooming")

Als Beispiel wurde weiterhin aus Polyamid 612 (Grilamid 2D20 / EMS Chemie) auf einem Einschneckenextruder ein einschichtiger Schlauch extrudiert, wobei aus dem extrudierten Schlauch durch konventionelles Streckblasformen mittels einer Ballonformanlage Ballone hergestellt wurden, wobei ähnliche Temperaturen wie für Schläuche aus PA12 oder PEBAX verwendet wurden (mit einem Blasdruck von ∼30bar). Mit den PA612-Ballonen wurden sodann PTCA-Katheter mit bestehenden Designs hergestellt, wobei die Eigenschaften der Ballone/Katheter (mit einem Durchmesser von ∼ 3,0 mm und einer Länge von 20 mm) insbesondere mit einem einschichtigen Hochdruckballonkatheter (PA12 als Ballonmaterial) verglichen wurden.

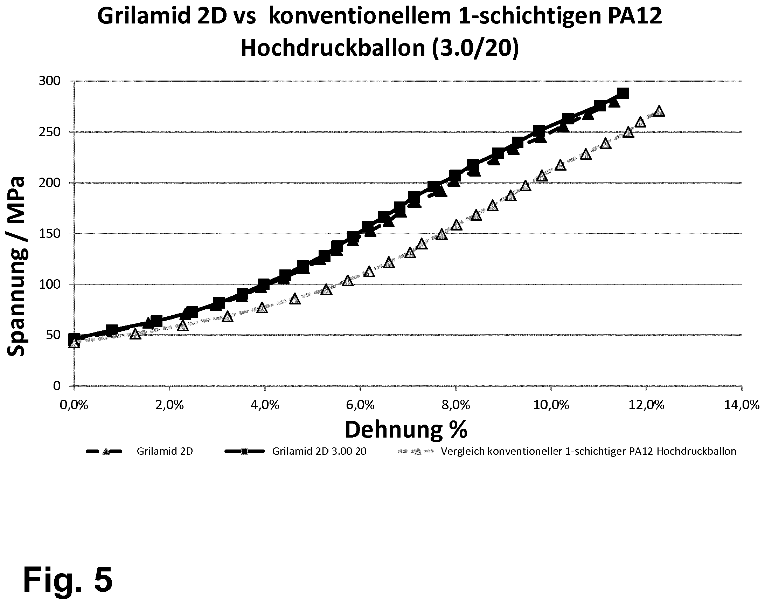

Hinsichtlich der Ballon-Compliance (Änderung des Ballondurchmessers in Abhängigkeit des Dilatations-Drucks), die in der Figur 5 gezeigt ist, ergibt sich dabei folgendes Bild. Die Compliance der Grilamid 2D20 Ballone ist im Bereich von ∼10 bar bis ∼20 bar vergleichbar zum einem bestehenden 1-schichten Hochdruck-Ballon (PA12 als Ballonmaterial) mit geringerer Ballonwandstärke.

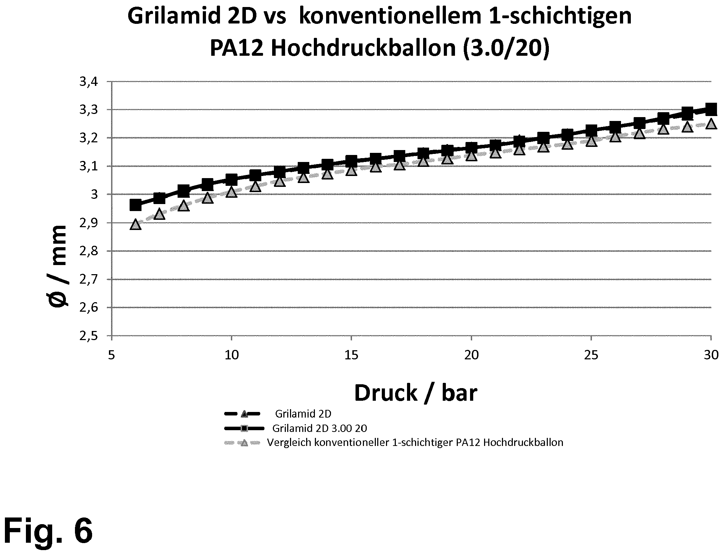

Die höhere Festigkeit von Grilamid 2D20 Ballonen im Vergleich zu einem bestehenden einschichtigen (PA12) Ballon zeigt sich auch im Spannungsdehnungs-Diagramm, das in der Fig. 6 gezeigt ist. Hieraus ergibt sich insbesondere, dass für die radiale Dehnung des Grilamid 2D20 Ballons eine höhere Spannung (Druck 1,2- bis 1,4-fach) benötigt wird, im Vergleich zu bestehenden 1-schichtigen PA12 Hochdruck-Ballonen.

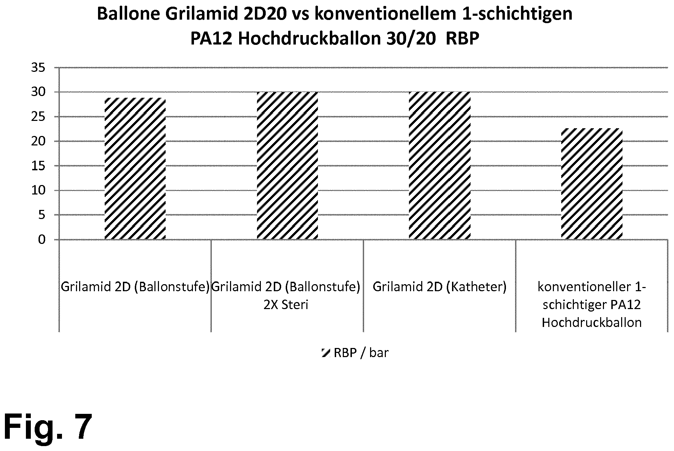

Im Hinblick auf den Rated Burst Pressure (RBP), d. h. den zulässigen Platzdruck, zeigt die Figur 7, dass dieser bei den Grilamid 2D-Komponenten höher liegt als beim Vergleichsballon (konventioneller PTCA Ballon 3,0/30: 17 bar).

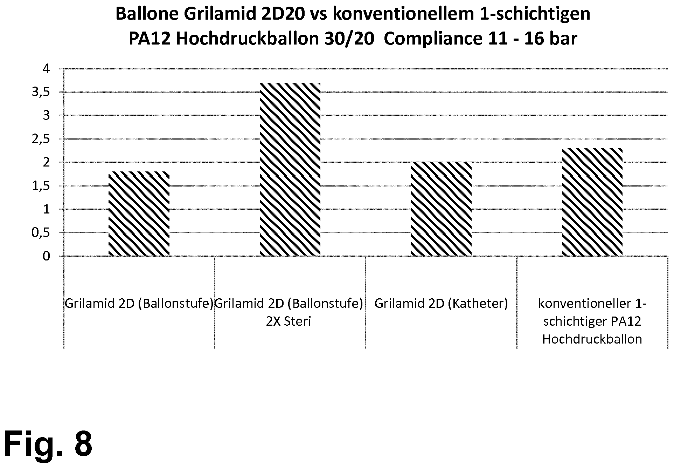

Auch die in der Figur 8 gezeigte Compliance zeigt ansprechende Werte der Grilamid 2D-Komponenten im Vergleich zum konventionellen PTCA Ballon: 5,0%.

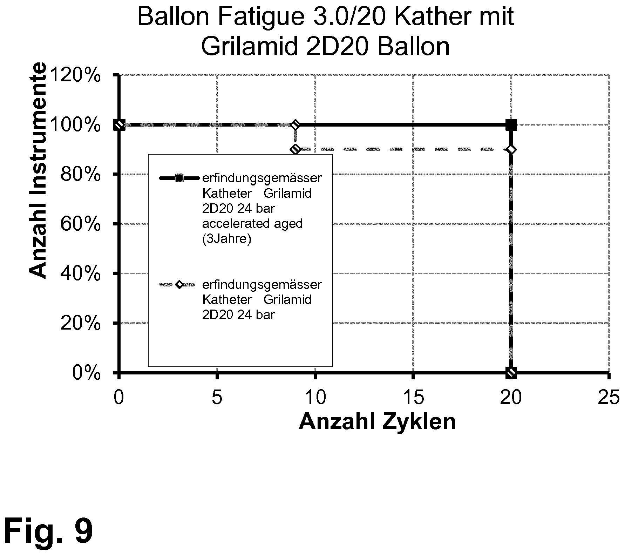

Wie ferner der Figur 9 zu entnehmen ist, die die Ballon Fatigue, d. h. die Beständigkeit des betreffenden Ballons bei mehrmaliger Belastung zeigt, besteht das Material Grilamid 2D den Ballon Fatigue Test, womit auch die funktionierende Verschweißung der Ballone mit dem Innen-, Außenschaft sowie der Spitze des Katheters nachgewiesen wurde.

Die Zusammenfassung der Ergebnisse mit Grilamid 2D20 (PA 612) Ballonen auf Ballon-Stufe (Eigenschaften der Ballone nach dem Ballonformen), ergibt folgendes Bild:

| Ballone | RBP | Wanddicke D /µm |
|---|---|---|
| Grilamid 2D20 Ballone | 30,0 bar | 19 |
| Vergleich einschichtiger Hochdruck-Ballon PA12-basierend | 24 bar | 20 |
| PEBAX MED Ballone | 25 bar | 24 |

Es lässt sich somit mittels des erfindungsgemäßen Ballonmaterials Grilamid 2D20 ein höherer RBP bei dünnerer doppelter Wandstärke im Vergleich zu PA12 oder PEBAX Ballonen erzielen. Die Balloneigenschaften bleiben auch nach zweimaliger Sterilisation erhalten.

Zusammenfassend ergibt sich auf der Katheter-Stufe (Balloneigenschaften am finalen sterilisierten Katheter), folgendes Bild (vgl. Fig.9):

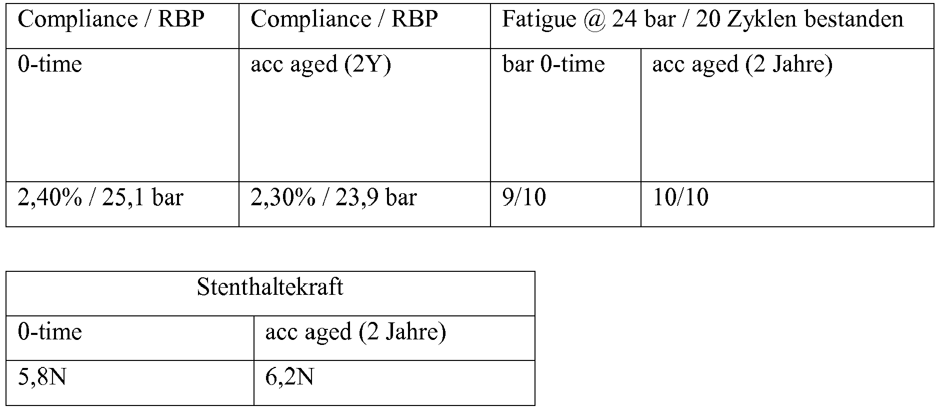

Es zeigt sich somit hier im Ergebnis eine geringe compliance mit 2,4%; die Ballon-Fatigue (20 Zyklen) konnte bei 24 bar nachgewiesen werden. Ferner ergibt sich eine ausreichende Stenthaltekraft.

## Patentansprüche

1. Ballon (1) für einen Ballonkatheter (2), wobei der Ballon (1) eine aufblasbare sowie einlagige Ballonhülle (10) aufweist, die einen Innenraum (11) umgibt und durch Befüllen des Innenraumes (11) mit einem Fluid (F) dilatierbar ist, die Ballonhülle (10) aus Polyamid 6.12 gebildet ist oder Polyamid 6.12 aufweist, **dadurch gekennzeichnet, dass** die Ballonhülle (10) eine Dicke im Verhältnis zum nominalen Ballondurchmesser im Bereich von 5,0 µm/mm bis 7,5 µm/mm aufweist.

2. Ballonkatheter (2), mit:
- einem Außenschaft (20) mit einem distalen Endabschnitt (20a),
- einen im Außenschaft (20) geführten Innenschaft (30), der mit einem distalen Endabschnitt (30a) über den distalen Endabschnitt (20a) des Außenschaftes (20) hinaussteht, sowie
- einem Ballon (1) gemäß Anspruch 1, wobei jenes Fluid (F) zum Dilatieren des Ballons (1) über den Außenschaft in den Innenraum der Ballonhülle einleitbar ist,
- wobei die Ballonhülle (10) mit einem proximalen Endabschnitt (10b) stoffschlüssig mit dem distalen Endabschnitt (20a) des Außenschaftes (20) verbunden ist, und wobei die Ballonhülle (10) mit einem distalen Endabschnitt (10a) mit dem distalen Endabschnitt (30a) des Innenschaftes (30) stoffschlüssig verbunden ist.

3. Ballonkatheter nach Anspruch 2, **dadurch gekennzeichnet, dass** der proximale Endabschnitt (10b) der Ballonhülle (10) mit dem distalen Endabschnitt (20a) des Außenschaftes (20) verschweißt ist, und/oder dass der distale Endabschnitt (10a) der Ballonhülle (10) mit dem distalen Endabschnitt (30a) des Innenschaftes (30) verschweißt ist.

4. Verfahren zur Herstellung einer Ballonhülle (10) für einen Ballonkatheter (2) nach einem der vorhergehenden Ansprüche, wobei eine mittels eines Fluids (F) dilatierbare Ballonhülle (10) des Ballons (1) aus einem Material geformt wird, bei dem es sich um Polyamid 6.12 handelt oder das Polyamid 6.12 aufweist, **wobei** zum Herstellen der Ballonhülle (10) das Material zu einem einlagigen, schlauchförmigen Rohling (100) extrudiert wird, der einen Innenraum (111) umschließt und sich entlang einer axialen Richtung (A) erstreckt.

5. Verfahren nach Anspruch 4, **wobei** der extrudierte Rohling (100) auf eine Temperatur im Bereich von 120°C bis 160°C, insbesondere 140°C, gebracht wird und radial zu der Form der Ballonhülle (10) expandiert wird.

6. Verfahren nach Anspruch 5, **wobei** das Expandieren ein Beaufschlagen des Innenraumes (111) des Rohlings (100) mit einem Druck im Bereich von 20 bar bis 50 bar, insbesondere 30 bar bis 35 bar, umfasst.

## Claims

1. A balloon (1) for a balloon catheter (2), the balloon (1) comprising an inflatable, single-layer balloon envelope (10), which surrounds an interior (11) and is dilatable by filling the interior (11) with a fluid (F), wherein the balloon envelope (10) consists of polyamide 6.12 or comprises polyamide 6.12, **characterized in that** the balloon envelope (10) has a ratio of the thickness of the balloon envelope to the nominal balloon diameter in the range from 5.0 µm/mm to 7.5 µm/mm.

2. A balloon catheter (2), comprising:
- an outer shaft (20) with a distal end section (20a);
- an inner shaft (30) guided in the outer shaft (20) and with a distal end section (30a) that projects out beyond the distal end section (20a) of the outer shaft (20); and
- a balloon (1) according to claim 1, wherein the fluid (F) for dilating the balloon (1) can be introduced through the outer shaft into the interior of the balloon envelope; wherein
- a proximal end section (10b) of the balloon envelope (10) is connected by material bonding with the distal end section (20a) of the outer shaft (20), and a distal end section (10a) of the balloon envelope (10) is connected by material bonding with the distal end section (30a) of the inner shaft (30).

3. A balloon catheter according to claim 2, **characterized in that** the proximal end section (10b) of the balloon envelope (10) is welded with the distal end section (20a) of the outer shaft (20) and/or **in that** the distal end section (10a) of the balloon envelope (10) is welded with the distal end section (30a) of the inner shaft (30).

4. A production process for a balloon envelope (10) for a balloon catheter (2) according to one of the preceding claims, wherein an inflatable balloon envelope (10) which is dilatable by a fluid (F) is formed from a material that consists of polyamide 6.12 or comprises polyamide, wherein for producing the balloon envelope (10) the material is extruded into a single-layer tubular blank (100) that surrounds an interior (111) and that extends in an axial direction (A).

5. A process according to claim 4, further comprising heating the extruded blank (100) to a temperature in the range from 120°C to 160°C, in particular 140°C, and radially expanding the blank into the shape of a balloon envelope (10).

6. A process according to claim 5, further comprising, during said radially expanding, applying a pressure in the range from 20 bar to 50 bar, in particular 30 bar to 35 bar to the interior (111) of the blank (100).

## Revendications

1. Ballonnet (1) pour un cathéter à ballonnet (2), le ballonnet (1) présentant une enveloppe de ballonnet (10), pouvant être gonflée ainsi qu'en une couche, qui entoure un espace intérieur (11) et qui peut être dilatée par le remplissage de l'espace intérieur (11) avec un fluide (F), l'enveloppe de ballonnet (10) est formée à base de polyamide 6.12 ou présente du polyamide 6.12, **caractérisé en ce que** l'enveloppe de ballonnet (10) présente une épaisseur en comparaison par rapport au diamètre de ballonnet nominal dans la plage de 5,0 µm/mm à 7,5 µm/mm.

2. Cathéter à ballonnet (2), doté:
- d'une tige extérieure (20) avec un segment d'extrémité distal (20a),
- d'une tige intérieure (30), menée dans la tige extérieure (30), qui dépasse vers l'extérieur avec un segment d'extrémité distal (30a) par-dessus le segment d'extrémité distal (20a) de la tige extérieure (20), ainsi que
- d'un ballonnet (1) selon la revendication 1, où le fluide (F), pour la dilatation du ballonnet (1), peut être introduit par le biais de la tige extérieure dans l'espace intérieur de l'enveloppe de ballonnet,
- où l'enveloppe de ballonnet (10) est reliée par complémentarité des matières avec un segment d'extrémité proximal (10b) au segment d'extrémité distal (20a) de la tige extérieure (20), et où l'enveloppe de ballonnet (10) est reliée par complémentarité des matières avec un segment d'extrémité distal (10a) au segment d'extrémité distal (30a) de la tige intérieure (30).

3. Cathéter à ballonnet selon la revendication 2, **caractérisé en ce que** le segment d'extrémité proximal (10b) de l'enveloppe de ballonnet (10) est soudé avec le segment d'extrémité distal (20a) de la tige extérieure (20), et/ou que le segment d'extrémité distal (10a) de l'enveloppe de ballonnet (10) est soudé avec le segment d'extrémité distal (30a) de la tige intérieure (30).

4. Procédé de fabrication d'une enveloppe de ballonnet (10) pour un cathéter à ballonnet (2) selon l'une des revendications précédentes, dans lequel une enveloppe de ballonnet (10) du ballonnet (1) pouvant être dilatée au moyen d'un fluide (F) est formée à partir d'un matériau, où il s'agit de polyamide 6.12 dans le cas du matériau ou présente du polyamide 6.12, **dans lequel,** pour la fabrication de l'enveloppe de ballonnet (10), le matériau est extrudé pour former une ébauche (100) en une couche en forme de tuyau qui entoure un espace intérieur (111) et s'étend le long d'une direction axiale (A).

5. Procédé selon la revendication 4, **dans** lequel l'ébauche (100) extrudée est amenée à une température dans la plage de 120 °C à 160 °C, notamment à 140 °C, et est expansée radialement pour donner la forme de l'enveloppe de ballonnet (10).

6. Procédé selon la revendication 5, **dans** lequel l'expansion comprend la soumission de l'espace intérieur (111) de l'ébauche (111) à une pression dans la plage de 20 bar à 50 bar, notamment de 30 bar à 35 bar.
